# EUROPEAN PATENT APPLICATION

(11) **EP 4 512 365 A1**
(43) Date of publication of application: **26.02.2025**
(21) Application number: 23792006.1
(22) Date of filing: 24.02.2023
(51) Int. Cl.: A61C 8/00, A61L 27/06, A61L 27/34, A61C 5/70

(54) **IMPLANT ABUTMENT ABSORBING EXTERNAL SHOCK AND MANUFACTURING METHOD THEREOF**

(30) Priority: 22.04.2022 KR 20220050064
(71) Applicant: Innoden Co., Ltd., Siheung-si, Gyeonggi-do 15057 (KR)
(72) Inventor: JANG, Cheon Seok, Ansan-si, Gyeonggi-do 15484 (KR)
(74) Representative: Cabinet Chaillot
(86) International application number: PCT/KR2023/002635
(87) International publication number: WO 2023/204424

(57) **Abstract**

The present invention relates to a technology related to an abutment for an implant, and more particularly, to an abutment for an implant capable of preventing a fixture, an abutment and/or abutment screw, or the like from being fractured due to repetitive occlusal force by integrally coating an elastomer on an outer surface of the abutment through a coating solution spraying and curing process during the manufacturing of the abutment for an implant made of titanium (Ti), and a manufacturing method thereof.

## Description

### [Technical Field]

The present invention relates to a technology related to an abutment for an implant, and more particularly, to an abutment for an implant capable of preventing fracture of a fixture, an abutment and/or abutment screw, or the like from being fractured due to repetitive occlusal force by integrally coating an elastomer on an outer surface of the abutment through a coating solution spraying and curing process during the manufacturing of the abutment for an implant made of titanium (Ti), and a manufacturing method thereof.

### [Background Art]

An implant is basically a fixture that is placed on an alveolar bone, an abutment that is fixed to the fixture and supports lateral or horizontal pressure in response to occlusal force applied during mastication, and a crown (artificial tooth) that restores an aesthetic beauty similar to a natural tooth by covering an upper portion of the abutment.

In general, a crown is manufactured in an optimized shape and size corresponding to a shape and size of the abutment, and then adhered to an upper surface of the abutment using an adhesive or the like. The crown is usually made of a hard material to mash food well during mastication. For example, as a material used for a crown, a dental metal or dental gold is cast and used, or the crown is manufactured by processing a zirconium oxide block through a CAD/CAM operation.

However, in the conventional implant structure, there is no component capable of absorbing the occlusal force like a periodontal ligament of natural teeth. For this reason, when the occlusal force is repeatedly applied to the crown, a problem may occur in that a fixture or an abutment and/or an abutment screw, or the like, which is a lower structure of the crown, may be repeatedly applied with the occlusal force and thus may be fractured.

### [Related Art Document]

### [Patent Document]

(Patent Document 1) KR 10-2304199 B1, 2021. 09. 14.
(Patent Document 2) KR 10-2304797 B1, 2021. 09. 15.
(Patent Document 3) KR 10-2132353 B1, 2020. 07. 03.
(Patent Document 4) KR 10-1924802 B1, 2018. 11. 28.

### [Disclosure]

### [Technical Problem]

Therefore, an object of the present invention provides an abutment for an implant absorbing external impact capable of preventing a fixture or an abutment and/or an abutment screw, etc., which are lower structures of a crown, from being fractured due to repetitive occlusal force, and a manufacturing method thereof.

In addition, an object of the present invention provides an abutment for an implant capable of stably supplying an abutment integrated with an elastomer by spraying a coating solution to an outer surface of the abutment and then curing the coating solution to coat the elastomer during the manufacturing of the abutment, and a manufacturing method thereof.

In addition, the present invention is not limited to the above-described purpose, and various objects may be additionally provided through technologies described through embodiments and claims to be described later.

### [Technical Solution]

According to an aspect of the present disclosure, an abutment for an implant absorbing external impact includes: an abutment body fastened and fixed to a fixture implanted in an alveolar bone; and an elastomer integrally coated on an outer surface of the abutment body to which an inner side surface of a crown is in contact to absorb the impact applied to the crown by repetitive occlusal force.

The abutment body may be made of a metal material, the elastomer may be formed by spraying a coating solution to the outer surface of the abutment body and curing the coating solution, the coating solution may use any one selected from the group consist of a liquefied synthetic resin material, a liquefied synthetic rubber material, a liquefied silicone material, and a liquefied silicone rubber material, and the elastomer may be formed to a thickness of 20 µm to 300 µm.

The abutment for an implant may further include: an abutment cap entering through an opening formed in the crown and screwed to the abutment body to fasten and fix the crown to the abutment body, in which the abutment cap may include a screw protrusion screwed into a screw groove part formed in the abutment body, and a head part formed on an upper portion of the screw protrusion and provided with a driver groove, and the elastomer may be integrally additionally coated to side and upper portions of the head or lower, side and upper portions of the head part other than the screw protrusion and the driver groove, in consideration of a contact area that is in direct contact with the crown according to a structure of the abutment cap.

The outer surfaces of the abutment body and the abutment cap may be surface-treated or formed with irregularities to increase bonding strength with the elastomer, and then may be coated with the elastomer.

A space in which the elastomer is accommodated may be formed on the inner side surface of the crown.

According to another aspect of the present disclosure, a method of manufacturing an abutment for an implant absorbing external impact includes: forming an abutment body fastened and fixed to a fixture implanted in an alveolar bone; and coating an elastomer integrally on an outer surface of the abutment body to which an inner side surface of a crown is in contact to absorb the impact applied to the crown by repetitive occlusal force.

The abutment body may be made of a metal material, in the forming of the elastomer, a coating solution may be sprayed to the outer surface of the abutment body to coat the elastomer base, and then the elastomer base may be cured to form the elastomer, the coating solution may use any one selected from the group consist of a liquefied synthetic resin material, a liquefied synthetic rubber material, a liquefied silicone material, and a liquefied silicone rubber material, and the elastomer may be formed to a thickness of 20 µm to 300 µm.

The abutment for an implant may further include: an abutment cap entering through an opening formed in the crown and screwed to the abutment body to fasten and fix the crown to the abutment body, in which the abutment cap may include a screw protrusion screwed into a screw groove part formed in the abutment body, and a head part formed on an upper portion of the screw protrusion and provided with a driver groove, and the elastomer may be integrally additionally coated to side and upper portions of the head or lower, side and upper portions of the head part other than the screw protrusion and the driver groove, in consideration of a contact area that is in direct contact with the crown according to a structure of the abutment cap.

The outer surfaces of the abutment body and the abutment cap may be surface-treated or formed with irregularities to increase bonding strength with the elastomer.

A space in which the elastomer is accommodated may be formed on the inner side surface of the crown.

### [Advantageous Effects]

As described above, according to an abutment for an implant absorbing external impact and a manufacturing method thereof of the present invention, by integrally forming an elastomer on an abutment body in contact with the crown during manufacturing of an abutment, it is possible to prevent an implant structure such as a fixture or an abutment and/or an abutment screw from being fractured due to repetitive occlusal force.

In addition, according to an abutment for an implant absorbing external impact and a manufacturing method thereof of the present invention, by spraying a coating solution to an outer surface of an abutment body in contact with a crown and curing the coating solution to form an elastomer, it is easy to manufacture the abutment integrated with the elastomer.

In addition, according to an abutment for an implant absorbing external impact and a manufacturing method thereof of the present invention, by integrally forming an elastomer on an abutment body and then supplying the elastomer during manufacturing of an abutment, there is no need to separately couple the elastomer to the abutment body during implantation, so it is possible to make an implant procedure provide simple and provide better convenience to an operator.

### [Description of Drawings]

FIG. 1 is a flowchart illustrating a method of manufacturing an abutment for an implant according to an embodiment of the present invention.
FIG. 2 is a flowchart illustrating a method of forming an elastomer illustrated in FIG. 1.
FIGS. 3 to 6 are cross-sectional views schematically illustrating the manufacturing method illustrated in FIGS. 1 and 2 for each process.
FIG. 7 is a cross-sectional view illustrating a method of manufacturing an abutment for an implant according to another embodiment of the present invention.
FIG. 8 is a cross-sectional view illustrating a method of manufacturing an abutment for an implant according to another embodiment of the present invention.
FIGS. 9 to 11 are diagrams schematically illustrating a method of manufacturing an implant according to another embodiment of the present invention.

### [Mode for Invention]

Various advantages and features of the present invention and methods accomplishing them will become apparent from the following description of embodiments with reference to the accompanying drawings. Also, like reference numerals designate like elements throughout this specification. In addition, each component illustrated in each figure may be excessively illustrated in size and shape, which is for convenience of description and is not intended to be limited. In addition, when described as "A and/or B," it may mean both A and B, or either A or B.

Hereinafter, embodiments of the present invention will be described in detail with reference to the accompanying drawings.

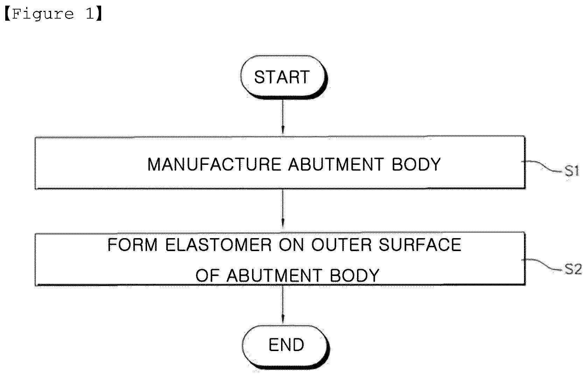

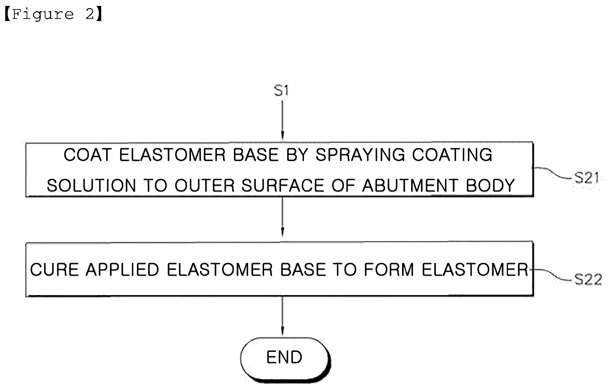

FIG. 1 is a flowchart illustrating a method for manufacturing an abutment for an implant according to an embodiment of the present invention, FIG. 2 is a flowchart illustrating a method of forming an elastomer illustrated in FIG. 1, and FIGS. 3 to 6 are cross-sectional views schematically illustrating the manufacturing method illustrated in FIGS. 1 and 2 for each process.

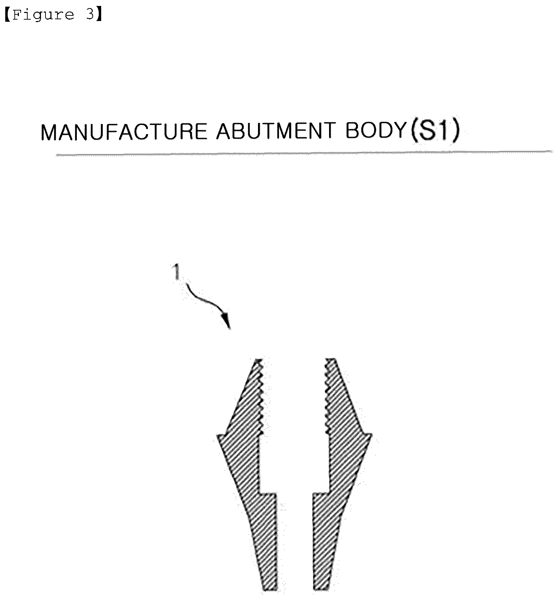

Referring to FIGS. 1 and 3, an abutment body 1 is manufactured (S1). In this case, the abutment body 1 may be made of, for example, titanium (Ti), and its shape and size are not limited to the structure illustrated in FIG. 3, and the abutment body 1 may be formed to have various shapes and sizes.

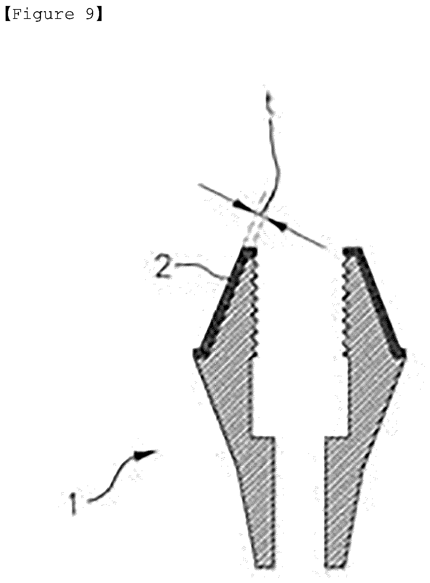

In FIG. 3, the abutment body 1 is formed in a two-piece structure in which the abutment screw 5 (see FIG. 9) is independently separated, which is an example. In the present invention, a one-piece structure in which the abutment screw is integrally formed is not excluded.

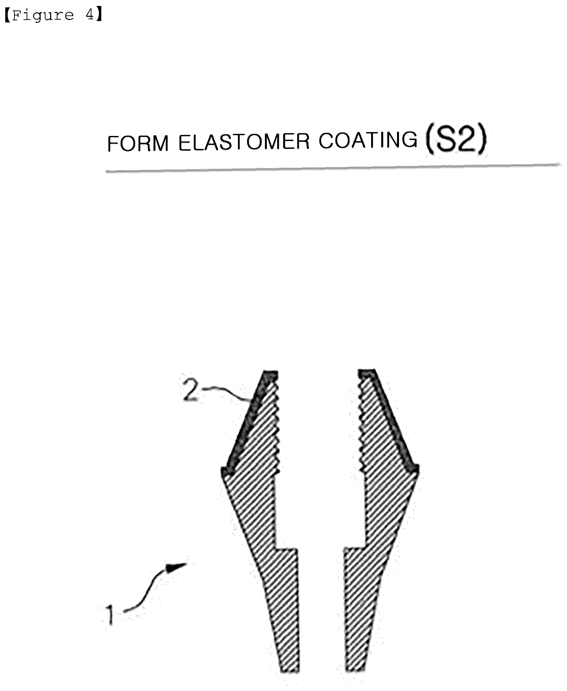

Subsequently, as illustrated in FIGS. 1 and 4, an outer surface of the abutment body 1 is coated with an elastomer 2 (S2).

The outer surface of the abutment body 1 may be surface-treated before coating the elastomer 2. The surface treatment may be performed by sand blasting or the like to increase the surface roughness of the abutment body 1 or to increase bonding strength of the elastomer 2 by forming irregularities on the outer surface.

The elastomer 2 is coated in the following way.

As illustrated in FIGS. 2, 5 and 6, an elastomer base 2a is coated by spraying a coating solution to the outer surface of the abutment body 1, and then the elastomer base 2a is cured to coat the elastomer 2.

The elastomer base 2a is coated on the outer surface of the abutment body 1 in contact with the crown.

As the coating solution, a material having excellent bonding strength to the abutment body 1 made of a metal material such as titanium is used. In addition, while having elasticity, it is possible to use a material having elasticity that can be coated on the outer surface of the abutment body 1 by a spraying method using a sprayer or spray nozzle. For example, any one selected from the group consisting of a liquefied synthetic resin material, a liquefied synthetic rubber material, a liquefied silicone material, and a liquefied silicone rubber material may be used.

The elastomer 2 is formed by spraying and coating the coating solution to the outer surface of the abutment body 1 using the spraying nozzle and then curing the coating solution. In this case, the elastomer 2 may be formed to a thickness of 20 µm to 300 µm in consideration of the fact that the movement of natural teeth by the occlusal force is 30 µm on average.

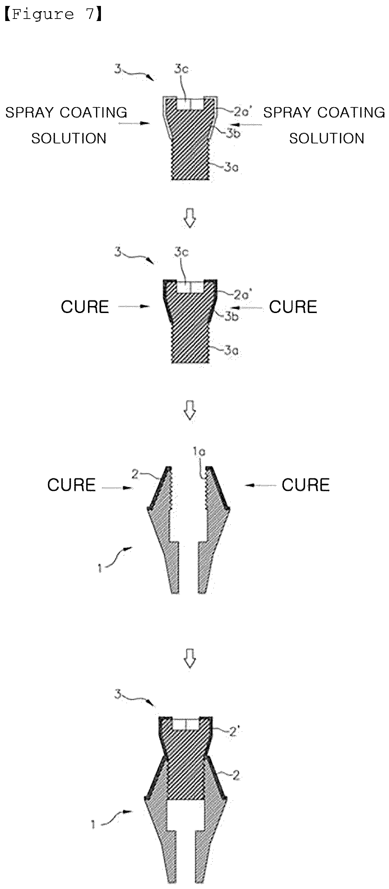

FIG. 7 is a cross-sectional view schematically illustrating a method of manufacturing an abutment for an implant according to another embodiment of the present invention, and is a diagram illustrating a process of integrally forming an elastomer on a tapered abutment cap and a process of bonding and fixing to an abutment body.

Referring to FIG. 7, in the abutment structure in which the crown is coupled and fixed to the abutment body 1 by the abutment cap 3, as the crown contacts not only the abutment body 1 but also the abutment cap 3, it is necessary to buffer a space between the abutment cap 3 and the crown.

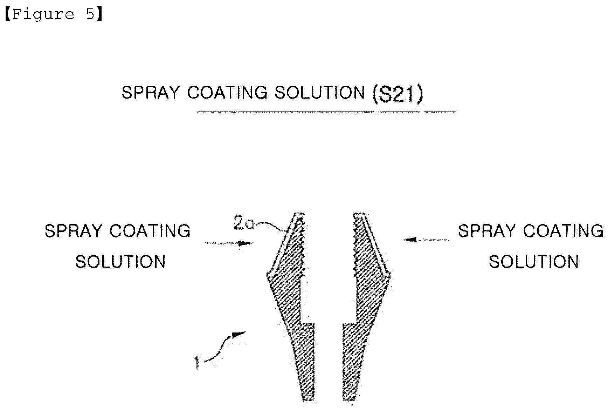

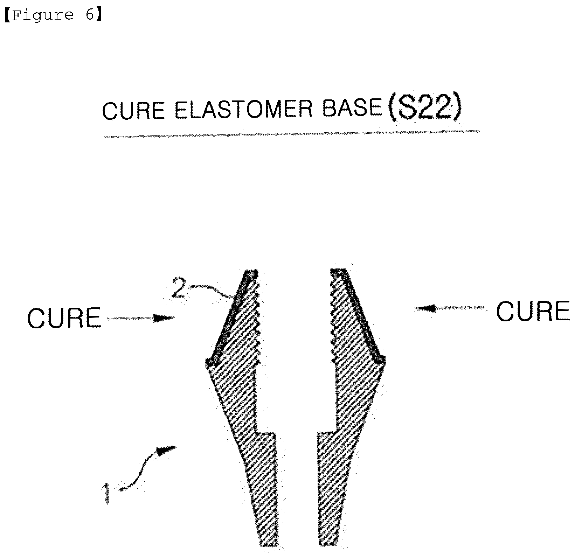

That is, as illustrated in FIGS. 5 and 6, when the crown is directly adhered to the abutment body 1 using an adhesive without using the abutment cap 3, the elastomer base 2a may be coated by spraying the coating solution only to the outer surface of the abutment body 1 in contact with the crown.

However, as illustrated in FIG. 7, in the structure in which the crown is coupled and fixed to the abutment body 1 by the abutment cap 3, when manufacturing the abutment cap 3 separately from the abutment body 1, an elastomer 2' may be formed on the outer surface of the abutment cap 3 through the coating solution spraying and curing process.

As illustrated in FIG. 7, the abutment cap 3 includes a screw protrusion 3a screwed to a screw groove part 1a of the abutment body 1 and a head part 3b formed at an upper portion of the screw protrusion 3a and having a driver groove 3c coupled to a driver tool and formed at a predetermined depth on the upper portion. The head part 3b is formed to have a larger width than the screw protrusion 3a, and protrudes to the upper portion of the abutment body 1 while the screw protrusion 3a is coupled to the screw groove part 1a of the abutment body 1.

The outer surface of the abutment cap 3 on which the elastomer 2' is coated may be surface-treated in the same way as the abutment body 1. The elastomer 2' is closely formed on the outer surface of the abutment cap 3 through the coating solution spraying and curing process. In this case, the portion where the elastomer 2' is formed may be changed according to the structure of the head part of the abutment cap 3. This is because when the structure of the head part of the abutment cap 3 is changed, the portion in contact with a crown 6 is also changed accordingly. Therefore, it may be formed in various portions according to the structure of the head part.

As illustrated in FIG. 7, when a side portion of the abutment cap 3 has a tapered (inclined) structure, as the tapered side portion directly contacts the crown 6, the elastomer 2' is formed on a side portion of the head part 3b and an upper portion where a resin filled in an opening 6a of the crown 6 contacts.

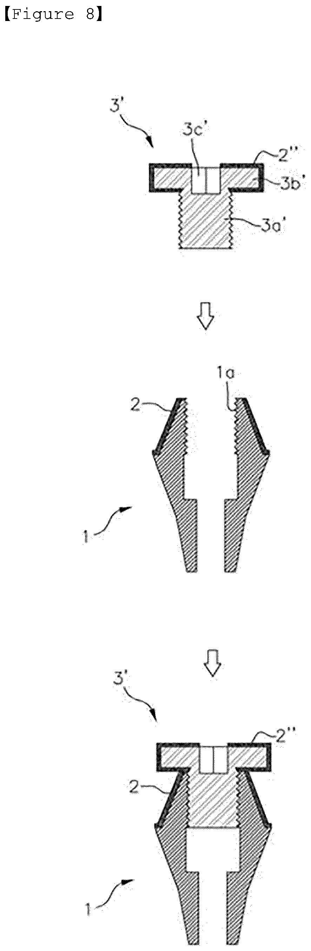

FIG. 8 is a cross-sectional view schematically illustrating a method of manufacturing an abutment for an implant according to another embodiment of the present invention, and is a diagram illustrating a process of coupling and fixing an abutment cap having a 'T' shape integrally formed with an elastomer to an abutment body.

As illustrated in FIG. 8, when an abutment cap 3' has a substantially 'T' -shaped structure, the abutment cap 3' is coupled and fixed to the abutment body 1, and then, is covered with the crown 6, so the lower portion, the side portion, and the upper portion (crown resin portion) of the head portion 3b' come into direct contact with the crown 6.

Therefore, in the abutment cap 3' structure having a 'T' shape, elastomer 2'' is formed on the lower, side, and upper portions of the head part 3b' except for the screw protrusion 3a' and the driver groove 3c'.

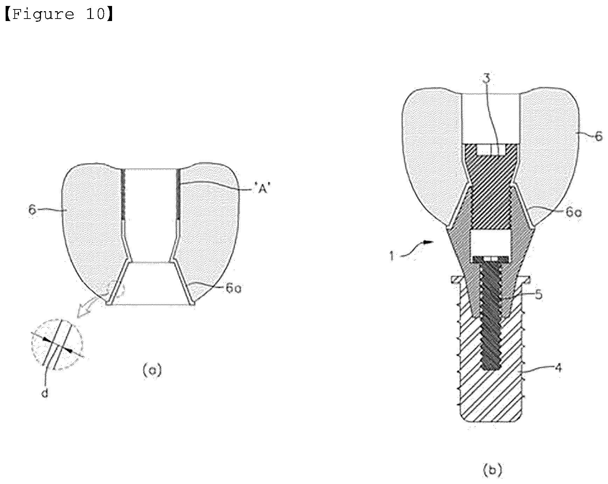

While being coupled to the abutment body 1, the side portion or the lower and side portions of the head parts 3b and 3b' come into contact with the inner side surface of the crown 6 (see FIG. 10), and the upper portions of the head parts 3b and 3b' come into contact with resin (not illustrated) filled in an opening 6b formed in the crown 6. Therefore, by forming the elastomer 2' and 2'' on all portions of the head part 3b that come into contact with the crown 6, even if an impact is applied to the crown 6 due to repetitive occlusal force, it may be stably absorbed.

As such, in the method of manufacturing an abutment for an implant method according to an embodiment of the present invention, when the abutment body 1 and the abutment caps 3 and 3' are manufactured in a manufacturing plant, the outer surface is coated with the elastomers 2, 2', and 2'' and supplied as a product in which the elastomers 2, 2', and 2'' is integrated.

FIGS. 9 to 11 are diagrams schematically illustrating a method of manufacturing an implant according to another embodiment of the present invention.

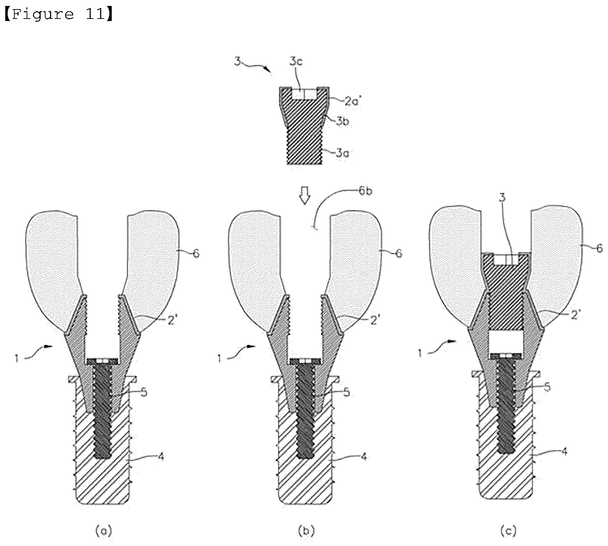

Referring to FIGS. 9 and 11, the abutment body 1 is fastened and fixed to the fixture 4 implanted in the alveolar bone (S2). In this case, the abutment body 1 may be fastened and fixed through the abutment screw 5.

In FIG. 9, as an example, the abutment body 1 is fastened to the fixture 4 by the abutment screw 5, but in the case of the one-piece structure in which the abutment screw is integrally formed with the abutment, the abutment may be directly fastened to the fixture.

Subsequently, as illustrated in FIG. 10, the crown 6, which is an artificial tooth, is manufactured to cover the abutment. In this case, the space 6a where the elastomer (2) will be formed on the inner side surface of the crown 6 in consideration of the shape and size of the elastomer 2 integrally formed on the abutment body 1 during the manufacturing of the crown 6 is formed in advance.

Of course, when the abutment cap 3 is applied, a space is provided for the elastomer 2' formed on the abutment cap 3 together with the elastomer 2 formed on the abutment body 1.

In the conventional implant structure in which the elastomers 2 and 2' are not installed, the inner area of the crown may extend to the space 6a and the hatched area 'A' as illustrated in FIG. 10A. However, in the embodiment of the present invention, considering the elastomers 2 and 2' to be installed between the crown 6 and the abutment body 1, the inner area may have an area reduced by the space 6a and the hatched area 'A'.

In the same abutment structure, the space 6a and the hatched area 'A', which are areas reduced compared to the conventional implant, may be changed according to the shape and size of the elastomers 2 and 2'. The space 6a has a shape corresponding to the shape of the elastomers 2 and 2'. In this case, a depth d of the space 6a may be formed to have a size equal to or slightly smaller than the thickness t of the elastomers 2 and 2'.

Subsequently, as illustrated in FIG. 11A, the crown 6 may be positioned on the abutment body 1 integrally formed with the elastomer 2, and then, as illustrated in FIGS. 11B and 11C, may be fastened and fixed using the abutment cap 3.

Subsequently, the opening 6a of the crown 6 is filled and embedded with a resin or the like later to complete the artificial tooth.

Hereinabove, the technical idea of the present invention has been specifically described in the embodiments, but is not limited to the embodiments and may be embodied in a variety of different forms, and is provided to fully inform those skilled in the art of the scope of the invention to which the present invention belongs, and the present invention is defined by the scope of the claims.

## Claims

1. An abutment for an implant absorbing external impact, comprising:
an abutment body (1) fastened and fixed to a fixture (4) implanted in an alveolar bone; and
an elastomer (2) integrally coated on an outer surface of the abutment body (1) to which an inner side surface of a crown (6) is in contact to absorb the impact applied to the crown (6) by repetitive occlusal force,
wherein a space (6a) in which the elastomer (2) is accommodated is formed on the inner side surface of the crown (6) .

2. The abutment for an implant of claim 1, wherein the abutment body (1) is made of a metal material,
the elastomer (2) is formed by spraying a coating solution to the outer surface of the abutment body (1) and curing the coating solution,
the coating solution uses any one selected from the group consist of a liquefied synthetic resin material, a liquefied synthetic rubber material, a liquefied silicone material, and a liquefied silicone rubber material, and
the elastomer (2) is formed to a thickness of 20 µm to 300 µm.

3. The abutment for an implant of claim 1, further comprising:
an abutment cap (3) entering through an opening formed in the crown (6) and screwed to the abutment body (1) to fasten and fix the crown (6) to the abutment body (1),
wherein the abutment cap (3) includes a screw protrusion (3a) screwed into a screw groove part (1a) formed in the abutment body (1), and a head part (3b) formed on an upper portion of the screw protrusion (3a) and provided with a driver groove (3c), and
the elastomer (2) is integrally additionally coated to side and upper portions of the head part (3b), or lower, side and upper portions of the head part (3b) other than the screw protrusion (3a) and the driver groove (3c), in consideration of a contact area that is in direct contact with the crown (6) according to a structure of the abutment cap (3).

4. The abutment for an implant of claim 3, wherein the outer surfaces of the abutment body (1) and the abutment cap (3) are surface-treated or formed with irregularities to increase bonding strength with the elastomer (2), and then are coated with the elastomer (2).

5. A method of manufacturing an abutment for an implant absorbing external impact, comprising:
forming an abutment body (1) fastened and fixed to a fixture (4) implanted in an alveolar bone; and
coating an elastomer (2') integrally on an outer surface of the abutment body (1) to which an inner side surface of a crown (6) is in contact to absorb the impact applied to the crown (6) by repetitive occlusal force,
wherein a space (6a) in which the elastomer (2') is accommodated is formed on the inner side surface of the crown (6) .

6. The method of claim 5, wherein the abutment body (1) is made of a metal material,
in the forming of the elastomer (2'), a coating solution is sprayed to the outer surface of the abutment body (1) to coat the elastomer base (2a), and then the elastomer base (2a) is cured to form the elastomer (2'),
the coating solution uses any one selected from the group consist of a liquefied synthetic resin material, a liquefied synthetic rubber material, a liquefied silicone material, and a liquefied silicone rubber material, and
the elastomer (2') is formed to a thickness of 20 µm to 300 µm.

7. The method of claim 5, wherein the crown (6) enters through an opening formed in the crown (6) and is fastened and fixed to the abutment body (1) by an abutment cap (3') screwed to the abutment body (1),
the abutment cap (3') includes a screw protrusion (3a') screwed to a screw groove part (1a) formed on the abutment body (1), and a head part (3b') formed on an upper portion of the screw protrusion (3a') and provided with a driver groove (3c'), and
the elastomer (2") is integrally additionally coated to side and upper portions of the head part (3b') or lower, side and upper portions of the head part (3b') other than the screw protrusion (3a') and the driver groove (3c'), in consideration of a contact area that is in direct contact with the crown (6) according to a structure of the abutment cap (3').

8. The method of claim 7, wherein the outer surfaces of the abutment body (1) and the abutment cap (3') are surface-treated or formed with irregularities to increase bonding strength with the elastomer (2").
